# EUROPEAN PATENT SPECIFICATION

(11) **EP 0 293 499 B1**
(45) Date of publication and mention of the grant of the patent: **01.09.1993**
(21) Application number: 87107895.2
(22) Date of filing: 01.06.1987
(51) Int. Cl.: A61N 1/05

(54) **Implantable multi-pole coaxial lead**
Implantierbare vielpolige koaxiale Leitung
Conducteur coaxial multipolaire implantable

(43) Date of publication of application: 07.12.1988
(73) Proprietor: Siemens-Elema AB, 171 95 Solna 1 (SE); SIEMENS AKTIENGESELLSCHAFT, 80333 München (DE)
(72) Inventor: Fahlström, Ulf, Dipl.-Ing., S-113 48 Stockholm (SE); Lekholm, Anders, Dr., S-161 39 Bromma (SE); Groothoff, Hermann, D-2908 Friesoythe (DE)

(56) References cited:
- EP-A- 0 162 178
- EP-A- 0 292 596
- CH-A- 656 313

## Description

The present invention relates to a multi-pole coaxial lead.

The demands on a multi-pole coaxial lead for use as part of an implanted electrode arrangement in i.e. heart pacemakers with a long life time are extremely high. A lead of this type must be i.e. body compatible, of low resistance to maintain energy consumption at a low level thereby increasing life time of pacemaker batteries, exhibit a high fatigue strength under repeated bending stresses, pliant in order to cause minimum disruptions to surrounding organs as well as stiff enough when pushed forward to enable an easy insertion at the clinic.

Moreover, these demands are at least in part contradicting as is obviously the case for pliant and low resistance requirements. In order to be pliant the conductors of the lead should essentially have a small diameter and in order to be of low resistance the conductors should have a great diameter.

The attempts to overcome these difficulties in the past are manifold. In contrast to a conventional coaxial lead DE-A-3 031 752 discloses a coaxial lead wherein the conductors are individually insulated and would into a single multi-pole helix. Although the diameter of this lead is reduced in comparison to a conventional lead with an inner and an outer helix of conductors separated from another by an insulating sheath and the conductors also being surrounded at the exterior of the lead by a further insulating sheath, the multi-pole helix necessarily has a steeper pitch and thus exhibits reduced flexibility. Further, the single insulating material requires a compromise between the electric properties and body compatibility for that material.

EP-A-0 092 798 presents a solution where the outer helix is a metal tape or tape shaped cord in order to reduce diameter and resistance while increasing flexibility.

A lead for i.e. catheters and electrical measurements in the medical field is examplified in DE-A-2 408 707 and comprises a multi-pole lead in which a multi-pole helix of axially separated conductors with a first insulating material is further insulated in such a way that the helix is comprised in the wall of a tube-formed second insulating material. The interior of the tube is a fluid conduit. Although this arrangement where the conductors and the first insulating material are embedded in the second insulating material enables a reduced diameter as well as food electrical insulating and body compatibility properties of this lead, the explicitly stated materials for the conductor, copper, and for the first insulating material, insulating paint, have however properties which make this lead unsuitable for implantation, especially intracardial implantation. The materials in a lead for intracardial implantation must withstand around 100,000 bendings a day, and consequently the lead must exhibit an extremely high fatigue strength. Further, if a stylet would be used at insertion, the walls of insulating material in the central opening of the lead could easily be penetrated.

EP-A-0 162 178 discloses a multi-pole coaxial lead for an electrode for stimulating body tissue having a plurality of conductors wound to a helix and each conductor being insulated by a first insulating material, the helix being arranged between an outer and an inner tube-formed insulating sheath; the inner opening of the inner insulating sheath comprises a helically wound coil being composed of a multi-pole arrangement of conductors.

EP-A-0 292 596, which was filed prior to the date of the present application and which was published after that date, discloses a multi-pole coaxial lead for an electrode for stimulating body tissue having a plurality of conductors wound to a helix and each conductor being insulated by a first insulating material, the insulated conductors being embedded in and axially separated from each other by a second, tube-formed insulating material, whereby the inner opening of said second material comprises a helically wound stylet guide coil.

It is an object of the present invention to provide an implantable multi-pole coaxial lead presenting an optimal compromise to the diverging requirements for stiffness, flexibility, fatigue strength and resistance discussed in the preceding paragraphs.

The above object is inventively achieved in the lead defined in claim 1.

The lead in claim 1 essentially defining a helix of individually insulated conductors embedded in the wall of the lead tubing and where the inner opening of the tubing comprises a helically wound stylet guide coil is advantageous in that it exhibits reduced diameter, good electrical and body compatibility properties in combination with a stylet guide member protective against penetration. Thereby the versatility of the lead is increased in that the stylet guide coil is composed of a multi-pole arrangement of conductors.

Preferably, to increase flexibility of the lead, at least one conductor is a multi-filament wire. Preferably, the filaments are helically wound.

In order to further improve lead properties at insertion all helically arrangements in the lead are wound in the same, i.e. left or right hand direction. This preferable embodiment corresponds to the habit of the surgeons to insert the lead in a way where the forward direction of the lead includes helical movement of the surgeon's hand in one direction, and withdrawal of the lead includes a movement of the surgeon's hand in the opposite direction. As it is preferable that the lead is flexible at withdrawal from the fixation tissue and stiff when approaching the fixation tissue, and several attempts may be required before an optimal electrode fixation is achieved, it is of great importance that this is an intrinsic property of the lead irrespective of stylet function. This is achieved by arranging all helixes in the same direction, viz., for right handed surgeons the helixes should be wound in the left hand direction and for left handed surgeons the helixes should be would in the right hand direction.

Other embodiments of the invention according to claims 5 to 7 are directed to e.g. preferable combinations of conductor and insulating materials.

In order that the invention may be more readily understood reference will now be made to the accompanying drawings.

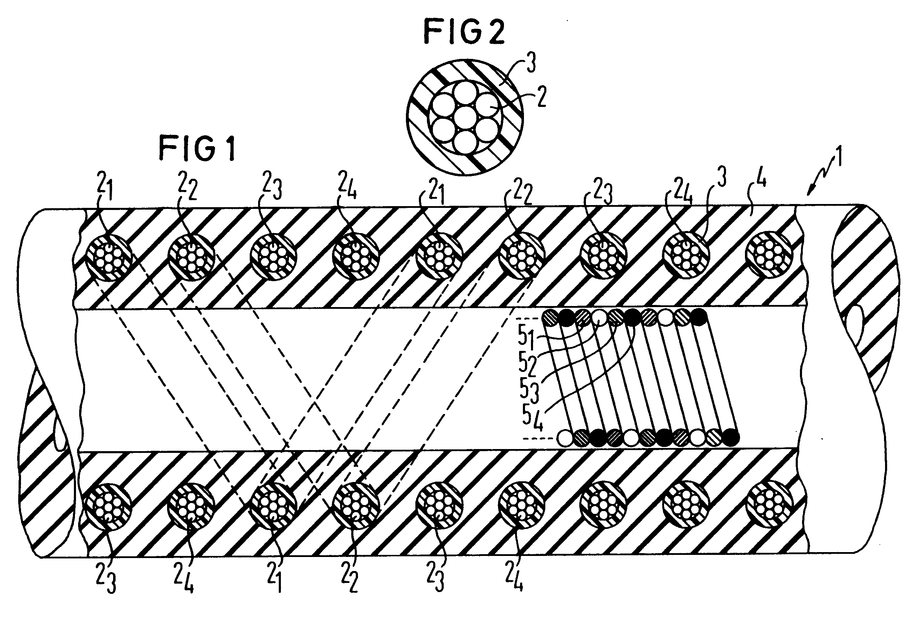

FIG 1 is an axial sectional view of a multi-pole coaxial lead constructed in accordance with the present invention illustrating a four-pole conductor arrangement embedded in an insulating tube of silicone rubber. The stylet guide coil is illustrated as a four-pole arrangement.

FIG 2 is a radial sectional view of a helically wound multi-filament conductor.

Referring now to the figures, an embodiment of the invention discloses a multi-pole lead 1 having outer helically wound conductors 2₁, 2₂, 2₃, 2₄ in a four-pole arrangement. The conductors 2₁ - 2₄ consist of multi-filament (e.g. refined steel) wire exhibiting high fatique strength under repeated bending stress. The filaments are all wound in the same direction. The conductors 2₁ - 2₄ are surrounded by a first insulating material 3 with good electric insulating properties, such as FEP (Fluorinated Ethylene Propylene) or PTFE (Polytetrafluorethylene). The conductors 2₁ - 2₄ are embedded in a second insulating material 4 with good body compatibility porperties, e.g. silicone rubber such as silastic Q7-4840 produced by Dow Corning Company. A central opening in the tube-formed second insulating material exhibits a stylet guide coil 5₁, 5₂, 5₃, 5₄. The guide coil conductors 5₁ - 5₄ may be comprised of MP 35 N, possibly in a multi-filament arrangement.

The conductors 2₁ - 2₄ and 5₁ - 5₄ may be individually identified by e.g. coloured insulation material, possibly combined with an X-ray contrast medium. By way of example, the guide coil could have an inner diamter of about 0,5 mmn and the lead could have an outer diameter of about 2,0 mm. The outer conductor helix is wound with a pitch of 2 to 4 mm, and the distance between the insulated conductors is around 0,2 mm. The tube-formed second insulating material 4, is produced in two steps, the first step comprising the extrusion of the inner part of the tube, and the second step comprising the extrusion of the outer part of the tube on the insulated outer conductors 2₁ - 2₄ wound on the inner part of the tube, so that the outer conductor helix is embedded in the integrated tube wall.

### Reference list

- 1: multi-pole lead
- 2: conductor
- 2₁, 2₂, 2₃, 2₄: four-pole conductor arrangement
- 3: first insulating material
- 4: second insulating material
- 5: Stylet guide coil
- 5₁, 5₂, 5₃, 5₄: multi-pole conductor

## Claims

1. A multi-pole coaxial lead (1) for an electrode for stimulating body tissue having a plurality of conductors (2₁, 2₂, 2₃, 2₄) wound to a helix and each conductor (2₁ - 2₄) being insulated by a first insulating material (3), the insulated conductors (2₁ - 2₄) being embedded in and axially separated from each other by a second, tube-formed, insulating material (4) whereby the inner opening of said second material (4) comprises a helically wound stylet guide coil being composed of a multi-pole arrangement of conductors (5₁, 5₂, 5₃, 5₄).

2. A lead according to claim 1, **characterized in that** at least one conductor (2₁ - 2₄, 5₁ - 5₄) is a multi-filament wire.

3. A lead according to claim 2, **characterized in that** the filaments are helically wound.

4. A lead according to any preceding claim, **characterized in that** all helically wound arrangement in the lead (1) are wound in the same direction.

5. A lead according to any preceding claim, **characterized in that** the first insulating material (3) is Fluorinated Ethylene Propylene (FEP) or Polytetrafluorethylene (PTFE) and that the second insulating material (4) is silicone rubber.

6. A lead according to any preceding claim in which the first insulating material (3) has an individual conductor identification.

7. A lead according to claim 6 in which said identification is an X-ray contrast medium, a colour or a combination thereof.

## Patentansprüche

1. Vielpolige koaxiale Leitung (1) für eine Elektrode zur Stimulierung von Körpergewebe mit einer Vielzahl von zu einer Wendel gewickelten elektrischen Leitern (2₁, 2₂, 2₃, 2₄), wobei jeder Leiter (2₁-2₄) mit einem ersten Isoliermaterial (3) isoliert und in einem zweiten, schlauchförmigen Isoliermaterial (4) eingebettet und durch dieses axial von den anderen Leitern getrennt ist und wobei die innere Öffnung des zweiten Isoliermaterials (4) eine wendelförmig gewickelte Spule zum Führen eines Mandrins aufweist, die sich aus einer Vielpolanordnung von elektrischen Leitern (5₁, 5₂, 5₃, 5₄) zusammensetzt.

2. Leitung nach Anspruch 1, **dadurch gekennzeichnet,** daß mindestens ein elektrischer Leiter (2₁-2₄, 5₁-5₄) ein mehrfädiger Draht ist.

3. Leitung nach Anspruch 2, **dadurch gekennzeichnet,** daß die Fäden wendelförmig gewickelt sind.

4. Leitung nach einem der vorhergehenden Ansprüche, **dadurch gekennzeichnet,** daß alle wendelförmig gewickelten Anordnungen in der Leitung (1) in der gleichen Richtung gewickelt sind.

5. Leitung nach einem der vorhergehenden Ansprüche, **dadurch gekennzeichnet,** daß das erste Isoliermaterial (3) fluoriertes Ethylen Propylen (FEP) oder Polytetrafluorethylen (PTFE) und das zweite Isoliermaterial (4) Silikongummi ist.

6. Leitung nach einem der vorhergehenden Ansprüche, bei der das erste Isoliermaterial (3) Mittel zum Identifizieren individueller elektrischer Leiter aufweist.

7. Leitung nach Anspruch 6, wobei die Identifizierungsmittel aus einem Röntgenstrahlkontrastmittel, einer Farbe oder einer Kombination von beiden bestehen.

## Revendications

1. Un câble coaxial multipolaire (1) pour une électrode destinée à la stimulation d'un tissu corporel, ce câble comprenant un ensemble de conducteurs (2₁, 2₂, 2₃, 2₄) enroulés en une hélice, et chaque conducteur (2₁ - 2₄) étant isolé par une première matière isolante (3), les conducteurs isolés (2₁ - 2₄) étant noyés dans une seconde matière isolante (4) de forme tubulaire, en étant séparés axialement les uns des autres par cette seconde matière isolante, la configuration étant telle que l'ouverture intérieure de la seconde matière isolante (4) contient une structure de guidage de stylet, enroulée en hélice, qui est constituée par une combinaison multipolaire de conducteurs (5₁, 5₂, 5₃, 5₄).

2. Un câble selon la revendication 1, caractérisé en ce qu'au moins un conducteur (2₁ - 2₄, 5₁ - 5₄) est un fil multifilament.

3. Un câble selon la revendication 2, caractérisé en ce que les filaments sont enroulés en hélice.

4. Un câble selon l'une quelconque des revendications précédentes, caractérisé en ce que toutes les structures enroulées en hélice dans le câble (1) sont enroulées dans la même direction.

5. Un câble selon l'une quelconque des revendications précédentes, caractérisé en ce que la première matière isolante (3) est de l'éthylène-propylène fluoré (ou FEP) ou du polytétrafluoréthylène (PTFE), et en ce que la seconde matière isolante (4) est du caoutchouc aux silicones.

6. Un câble selon l'une quelconque des revendications précédentes, dans lequel la première matière isolante (3) comporte une identification individuelle des conducteurs.

7. Un câble selon la revendication 6, dans lequel l'identification consiste en une substance de contraste pour rayons X, en une couleur ou une combinaison de celles-ci.
